# EUROPEAN PATENT APPLICATION

(11) **EP 4 360 663 A1**
(43) Date of publication of application: **01.05.2024**
(21) Application number: 22878247.0
(22) Date of filing: 05.09.2022
(51) Int. Cl.: A61L 2/10, A61L 9/20, C02F 1/32

(54) **FLUID-CLEANING CIRCULATOR**

(30) Priority: 05.10.2021 JP 2021163831
(71) Applicant: Reiz Co., Ltd., Kitakyushu-shi, Fukuoka 804-0065 (JP); Fukuoka Prefecture, Fukuoka-shi, Fukuoka 812-8577 (JP)
(72) Inventor: ISHIDA Shinji, Kitakyushu-shi Fukuoka 804-0065 (JP); TANAKA Masatoshi, Kitakyushu-shi Fukuoka 807-0831 (JP)
(74) Representative: Algemeen Octrooi- en Merkenbureau B.V.
(86) International application number: PCT/JP2022/033212
(87) International publication number: WO 2023/058376

(57) **Abstract**

With a fluid cleaning circulator having a hollow circular pipe in which a passage of a fluid is formed in a spiral shape, harmful microorganisms in the fluid are deactivated by ultraviolet light irradiation. Surface processing that reflects ultraviolet light is applied to an inner surface of the hollow circular pipe. A radiation prevention means that prevents radiation of an ultraviolet light from both ends of the hollow circular pipe to the outside is provided. An ultraviolet light LED element is provided on an inner wall of the hollow circular pipe only within a distance of approximately 1/2 of a flow passage length of the hollow circular pipe from a fluid inlet side end portion of a closed container of the hollow circular pipe.

## Description

### Technical Field

The present invention relates to a safe and hygienic fluid cleaning circulator that deactivates harmful microorganisms such as viruses in a fluid regarding air and water serving as a fluid of surroundings by LED ultraviolet light irradiation without leaking an ultraviolet light harmful to the human body to the outside.

### Background Art

In recent years, there has been a tendency that powerful viruses which possess unprecedented dangers such as SARS and COVID-19 are being spread by human beings. In order to avoid infections from these viruses and to maintain the safety of life and livelihood, there is a need to clean the surroundings.

As preventive methods to be taken as individual activities, wearing masks, gargling, hand washing, avoiding closed spaces, crowded places, and close-contact settings, etc., are considered to be effective means. However, there are limitations to what can be prevented only by individual activities, and in addition to these, cleaning air and water which are surrounding environments themselves is demanded as a further hygienic means.

Non-patent Literature 1 describes a UV-C irradiation device in which a UV-C sterilization lamp is arranged in the center and enclosed by a spiral passage having a rectangular cross-section, and shows a test result that the UV-C irradiation device is effective for deactivation of COVID-19. However, there are several points to be improved even in this UV-C irradiation device.

First, by use of the UV-C sterilization lamp as an ultraviolet light source, the UV-C irradiation device has a characteristic that electric power consumption is high and not only an ultraviolet light of a specific wavelength but also wavelengths in the vicinity of the wavelength are broadly emitted. Thus, a large energy source is required to drive, and there is little freedom in installing the UV-C irradiation device in any place desired.

Next, the ultraviolet light emitted by the UV-C sterilization lamp in the center passes through the spiral passage of a transparent material, and abuts with an inner wall of a casing. Surface processing is applied to the inner wall of the casing, and the ultraviolet light is multiple reflected inside the casing. Although the ultraviolet light reflected and returned to the center abuts with the UV-C sterilization lamp, the UV-C sterilization lamp itself does not have a reflection function. Thus, efficient multiple reflection cannot be expected.

### Citation List

### Patent Literature

Non-patent Literature 1: "Deactivation Test Result of Corona Virus Infectious Disease-2019 (SARS-CoV-2) by Dr. AiR (Michiwaki UV-C irradiation device) implemented in the Kitasato Institute" (NejiLaw inc.) URL: http://www.nejilaw.com/pdf/NejiLaw_PressRelease_20210617.pdf

### Summary of Invention

### Technical Problem

An object of the present invention is to solve all the problems the conventional art has as described above, that is, high electric power consumption, inefficiency by ultraviolet light irradiation of wavelengths other than a required wavelength, low degree of freedom in installation place, low utilization efficiency of the generated ultraviolet light due to low reflection efficiency, possibility of leakage of an ultraviolet light harmful to the human body to the outside of the device, etc., and to sufficiently and safely exert effects equal to or more than the effect of deactivating harmful viruses proven by the conventional art.

### Solution to Problem

The invention described in Claim 1 is a fluid cleaning circulator that deactivates harmful microorganisms in a fluid by ultraviolet light irradiation, wherein a hollow circular pipe formed in a spiral shape inside a closed container is provided, both ends of the hollow circular pipe are fixed to an inner wall of the closed container, and holes for a fluid inlet and a fluid outlet for allowing the fluid to flow through the hollow circular pipe are formed in the closed container, surface processing that reflects ultraviolet light is applied to an inner surface of the hollow circular pipe, and a radiation prevention means that prevents radiation of an ultraviolet light from the fluid inlet and the fluid outlet to the outside is provided, an ultraviolet light LED element is provided on an inner wall of the hollow circular pipe only within a distance of approximately 1/2 of a flow passage length of the hollow circular pipe from an end portion of the hollow circular pipe on the fluid inlet side, and an interior of the hollow circular pipe is irradiated by light emission by the ultraviolet light LED element.

The invention described in Claim 2 is the fluid cleaning circulator according to Claim 1, wherein the ultraviolet light to be irradiated is UV-C, and has a wavelength of 250 to 280 nm.

First, an ultraviolet light LED serving as an ultraviolet light generation source is used. With a decrease in necessary drive electric power, consequent high degree of freedom in installation place, and use of an element specialized for a wavelength having the most virucidal effect, it is possible to achieve the object with low electric power and high efficiency.

Next, by irradiating the ultraviolet light from the ultraviolet light LED arranged on the inner wall of the spiral hollow circular pipe having a small inner diameter and a long passage into the hollow circular pipe, most of the radiated ultraviolet light has an increased incidence angle with respect to the inner surface of the hollow circular pipe, and in combination with the processed inner surface to reflect the ultraviolet light, the ultraviolet light generated from the LED contributes to deactivation of viruses at maximum.

That is, since the ultraviolet light is reflected on the inner surface of the hollow circular pipe to which the surface processing that reflects ultraviolet light is applied, the ultraviolet light moves toward the suction port side and the discharge port side of the hollow circular pipe (hereinafter, simply referred to as the "both end sides") while attenuating due to repeated multiple reflection. When a reflection ratio of the inner surface of the hollow circular pipe is high, the attenuation is decreased. Thereby, (1) it is possible to fill the passage (internal space of the hollow circular pipe) with the ultraviolet light while performing multiple reflection, and deactivate all viruses and bacteria. In addition, (2) since there is an enormous number of multiple reflections, the ultraviolet light attenuates as it moves toward both end sides of the hollow circular pipe, and it is possible to prevent leakage of the ultraviolet light (leaked light) in the vicinity of both ends of the hollow circular pipe.

Further, by arranging the ultraviolet light LED only within the distance of approximately 1/2 of the flow passage length of the hollow circular pipe from the end portion of the hollow circular pipe on the fluid inlet side, that is, a position of about a half of the length of the flow passage formed by the inner wall of the hollow circular pipe, through which the fluid flows, the ultraviolet light reflects and propagates toward both the upstream side and the downstream side of the passage. The longer a propagation distance becomes, the more the ultraviolet light attenuates. Thus, the ultraviolet light attenuates to such a safe extent in the vicinity of both ends of the hollow circular pipe. Further, by providing the radiation prevention means that prevents the radiation of the ultraviolet light from both ends of the hollow circular pipe to the outside, the ultraviolet light is prevented from leaking out to the outside and safety is ensured.

The distance of approximately 1/2 of the flow passage length of the hollow circular pipe from the end portion of the hollow circular pipe on the fluid inlet side may be a position where, since the interior of the hollow circular pipe is filled with the ultraviolet light by multiple reflection of the ultraviolet light, in a case where the ultraviolet light LED is installed in one place, the interior of the hollow circular pipe can be filled with the ultraviolet light, and is not necessarily limited only to the distance of 1/2 of the flow passage length of the hollow circular pipe from the end portion of the hollow circular pipe on the fluid inlet side.

For example, the radiation prevention means includes a method of making an inner surface in the vicinity of both ends of the hollow circular pipe with a material that does not reflect an ultraviolet light, a method of applying surface processing that does not reflect ultraviolet light to the inner surface in the vicinity of both ends of the hollow circular pipe, etc.

### Effect of Invention

As described above, the fluid cleaning circulator of the present invention can solve all the problems of the conventional art, with low electric power consumption and degree of freedom in installation, an efficient virus deactivation effect by the LED specialized for the ultraviolet light of the specific wavelength, maximized utilization of the irradiated ultraviolet light by multiple reflection which is realized by inner surface processing, sufficient attenuation by the distance from the LED installation place to both ends of the hollow circular pipe, and prevention of leakage of the harmful ultraviolet light by non-reflection surface processing in the vicinity of both ends of the hollow circular pipe.

In addition, by adjustment of an output of the ultraviolet light LED element to be used and a flow speed by a fan, it is possible to adjust a flow rate which can be processed per time, and the present invention is maintenance-free as a filter, etc., is not used.

### Brief Description of Drawings

[FIG. 1] FIG. 1(a) is an outer appearance image view showing a structure of a fluid cleaning circulator according to an embodiment of the present invention; and FIG. 1(b) is an image view showing an internal structure of the fluid cleaning circulator according to the embodiment of the present invention.
[FIG. 2] FIG. 2 is a graph showing a result of simulation of an exposure light amount per 1/4 of an elliptic circumference in the fluid cleaning circulator according to the embodiment of the present invention.
[FIG. 3] FIG. 3 is a graph showing a result of simulation of an integrated exposure light amount in the fluid cleaning circulator in the fluid cleaning circulator according to the embodiment of the present invention.

### Description of Embodiments

Hereinafter, preferred modes for implementing the present invention will be specifically described. However, these do not limit the scope of the present invention.

A fluid cleaning circulator according to an embodiment of the present invention (hereinafter, simply referred to as the "circulator") can deactivate harmful microorganisms in a fluid by ultraviolet light irradiation. The circulator has a closed container 11, a hollow circular pipe 12 in which a passage of the fluid provided inside the closed container 11 is formed in a spiral shape, and an ultraviolet light LED element provided on an inner wall surface of the hollow circular pipe 12.

A material of the hollow circular pipe 12 is not particularly limited, and the hollow circular pipe 12 may be made of metal or resin. With weight, strength, processing property to a spiral shape, cost, ease of processing for reflection or non-reflection of an ultraviolet light by an inner surface, etc., in mind, a favorable material is selected.

Although a hollow part of the hollow circular pipe 12 is preferably a perfect circle in a cross-sectional view, an ellipse can also be applied. When the hollow circular pipe 12 is a perfect circle in a cross-sectional view, the hollow circular pipe 12 can contribute to efficient reflection of the ultraviolet light. In addition, a smaller inner diameter is preferable from the point that a passage length of the hollow circular pipe 12 can be long. However, when the inner diameter is too small, a flow passage cannot be ensured, and the deactivation and sterilization effect is lowered. In addition, multiple reflection of the ultraviolet light increases too much, the attenuation ratio of the ultraviolet light over an interior of the flow passage is increased, and consequently the deactivation and sterilization effect is lowered.

With a relatively thin outer diameter of the hollow circular pipe 12, the passage length in the same volume can be long, an in-pipe staying time of the fluid, a distance of transmission of the ultraviolet light from the ultraviolet light LED element to be described later, and sufficient attenuation in the vicinity of an outlet and an inlet are ensured, and processing to a spiral shape becomes easy. Further, density per section of radiation flux generated from the ultraviolet light LED element is increased and uniformity is enhanced. Thus, when reaching both ends of the hollow circular pipe 12 by multiple reflection, the ultraviolet light attenuates and prevention of leakage of the ultraviolet light becomes easy.

According to the size of the circulator, the size of the hollow circular pipe 12 should be appropriately designed. However, it should be noted that, when the hollow circular pipe 12 is excessively thin, flow passage resistance is increased, and the size of an ultraviolet light LED element which can be mounted is limited.

By arranging the hollow circular pipe 12 in a spiral shape (that is, making a flow passage formed by the hollow part of the hollow circular pipe 12 a spiral shape), a rectilinear propagation of the light in the hollow circular pipe 12 is impaired as little as possible, while it is possible to ensure a sufficient passage length of the hollow circular pipe 12 within a range covered by the same volume. According to an installation place, for a thin place, by shortening a spiral radius of the hollow circular pipe 12 and accommodating in a thin and long tubular container, or for a place where a height cannot be ensured, by making a spiral shape of the hollow circular pipe 12 a substantially elliptic shape and accommodating in an elliptic tubular container having high ellipticity, degree of freedom in outer appearance shape is high. Even with a change in the shape, when the inner diameter of the hollow circular pipe 12 and a flow passage length are equal to each other, the cleaning ability of those is basically unchanged.

The inner surface of the hollow circular pipe 12 is processed with a nearly mirror finish by deposition, plating, adhering, etc., of metal. It is noted that a type of the metal does not particularly matter as long as the metal is a material having a high ratio of reflecting the ultraviolet light such as aluminum, but may generally be aluminum deposition, aluminum plating, adhering of an aluminum reflection sheet, gold plating, silver plating, zinc plating, tin plating, etc. In the present embodiment, the adhering of the aluminum reflection sheet or the aluminum deposition is adopted.

In addition, in order to prevent the leakage of the ultraviolet light from both ends of the hollow circular pipe 12 to the outside, parts in the vicinity of both ends of the hollow circular pipe 12 (length of approximately 2 to 5% of the entire length from the end portion) are made of a material that does not reflect the ultraviolet light, or surface processing that does not reflect ultraviolet light is applied. In the present embodiment, plating processing is not performed, but by applying a black material, the material that can prevent radiation of the ultraviolet light to the outside of the hollow circular pipe 12 is adopted. In addition to this, it is also possible not to perform surface processing but use a black resin material that does not reflect the ultraviolet light.

A range of the surface processing that does not reflect ultraviolet light is the vicinity of both ends of the hollow circular pipe 12, and includes at least a range which is visible at the time of looking into an opening portion from the outside. However, it is preferable to perform the surface processing to a range which is equal to or more than the range to allow leeway to some extent, specifically, a range of about a semiperimeter of the spiral. It is preferable to perform the surface processing that reflects ultraviolet light to all the other parts of the passage.

The ultraviolet light LED is arranged within a distance of approximately 1/2 of the flow passage length of the hollow circular pipe from a suction port 11A side end portion of the hollow circular pipe 12, that is, at a position of a half of the length of the flow passage of the fluid formed by an inner wall of the hollow circular pipe 12.

A commercially available ultraviolet light LED element may be used. In addition, a newly designed ultraviolet light LED element may be used. In consideration of the inner diameter of the hollow circular pipe 12, the passage length, a flow speed by the rotation number of a fan, etc., an ultraviolet light LED element is appropriately used which properly has an emission wavelength of the element, an emission spectrum characteristics, emission strength, and the number of elements to be used so that a sufficient cleaning effect is exerted between the suction port 11A and a discharge port 11B. The LED element is installed so as to be fitted into the opening portion opened in the vicinity of the center of the passage of the hollow circular pipe 12 and there is a need to pay attention so that the ultraviolet light and the fluid inside are not leaked out to the outside.

As an energy source to be used for driving the ultraviolet light LED element and the fan, an AC power source, a DC power source, etc., can be freely adopted. It is considered that, when an installation place is an interior of a car, a DC 12V is used, and when the installation place is outdoors, the voltage is converted into DC 12V by an AC adapter, or a 12V battery is used.

Regarding a wavelength of the ultraviolet light to be used, a wavelength highly effective for deactivating viruses, etc., is used. An ultraviolet light of 250 to 280 nm called UV-C is useful in the point of having a strong sterilization ability, but attention is required to handle UV-C in the point that the human body may be badly affected.

Both ends of the hollow circular pipe 12 are fixed to an inner wall of the closed container 11. In addition, the suction port 11A and the discharge port 11B are formed in the closed container 11 so that the fluid flows through the hollow circular pipe 12.

That is, the number of holes formed in the closed container 11 is two, and an opening shape of the holes is the same as a shape of the suction port 11A side end portion and a discharge port 11B side end portion of the hollow circular pipe 12 (cross-sectional shape of the hollow part in the direction orthogonal to the longitudinal direction of the hollow circular pipe 12 in both end portions of the hollow circular pipe 12). Then, both ends of the hollow pipe 12 are fixed to the inner wall of the closed container 11 so that the suction port 11A and the discharge port 11B of the closed container 11 overlap both ends of the hollow circular pipe 12 (so that part of or all of the hollow part of the hollow circular pipe 12 is not covered by the closed container 11).

The fan for allowing the fluid to flow through the flow passage of the hollow circular pipe 12 is provided in this circulator.

The fan may be installed in any of both the vicinity of the suction port 11A side of the hollow circular pipe 12 and the vicinity of the discharge port 11B side. A small and light fan of a DC motor that can be driven with low voltage can be utilized at low cost. The rotation number of the fan is determined by calculating so that the ultraviolet light flows at a required flow speed, that is, taking a staying time required for deactivating viruses, etc., in the volume determined from a cross-sectional area and the flow passage length of the hollow circular pipe 12. A diameter of the fan is preferably similar to a diameter of the hollow circular pipe 12, however, a fan having a large diameter can be rotated at low speed in order to reduce noise. In this case, a funnel-shaped member may be placed between the hollow circular pipe 12 and the fan.

In a case where the fluid is water, it is also possible to allow the water to flow in by pressure of running water, etc., in place of the fan.

Hereinafter, description will be given by using an example.

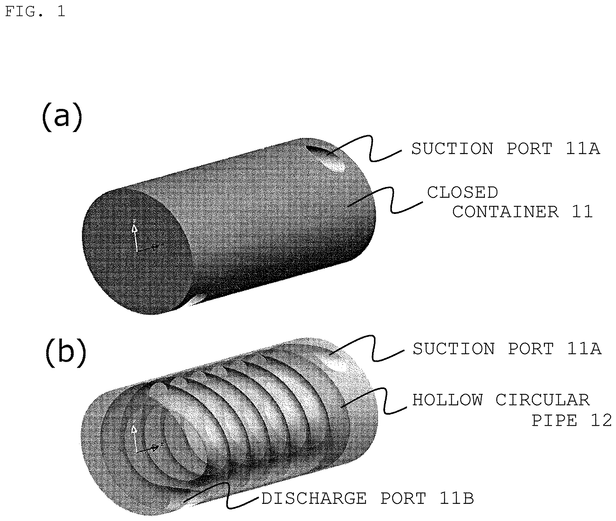

The UV-C LED fluid cleaning circulator was designed as in FIG. 1 and formed with the diameter of the fan of 20 mm, the diameter of the hollow circular pipe 12 of 20 mm, and a path shape of an elliptic path with a long axis radius of 64 mm and a short axis radius of 44 mm with an angle of 7°. A shape of the entire spiral became an elliptic tube shape having a width of 150 mm, a height of 110 mm, and a length of 200 mm. The flow passage length of the hollow circular pipe 12 was roughly estimated as 2,588.0 mm. Regarding an interior of the pipe, an elliptic semiperimeter in the vicinity of the outlet and the inlet was coated in black, and the other part was metal-plated.

A structure in which this UV-C LED package was installed on a back surface in the center of the spiral passage and the ultraviolet light was irradiated toward the interior of the pipe was taken.

### (Simulation of Exposure Light Amount per 1/4 of Elliptic Circumference)

An exposure light amount per 1/4 of an elliptic circumference of the spiral of the hollow circular pipe 12 was determined by simulation.

On the assumption that there is a light receiving surface for each 1/4 section of the elliptic circumference of the spiral from the suction port 11A side end portion of the hollow circular pipe 12 to the discharge port 11B side end portion, the exposure light amount for each light receiving surface was calculated.

When a roughly estimated value of the 1/4 section of the elliptic circumference of the flow passage is 86.3 mm and the flow speed is v m/s, the staying time is "86.3 / (v × 1,000)." An average value of average irradiance per each 1/4 section of the elliptic circumference is Ee, ave, i-j. The light amount exposed by the fluid cleaning circulator is determined by "SUM (Ee, ave, i-j × 86.3 / (v × 1,000))." At this time, the calculation was carried out given that the flow speed was 1 m/s.

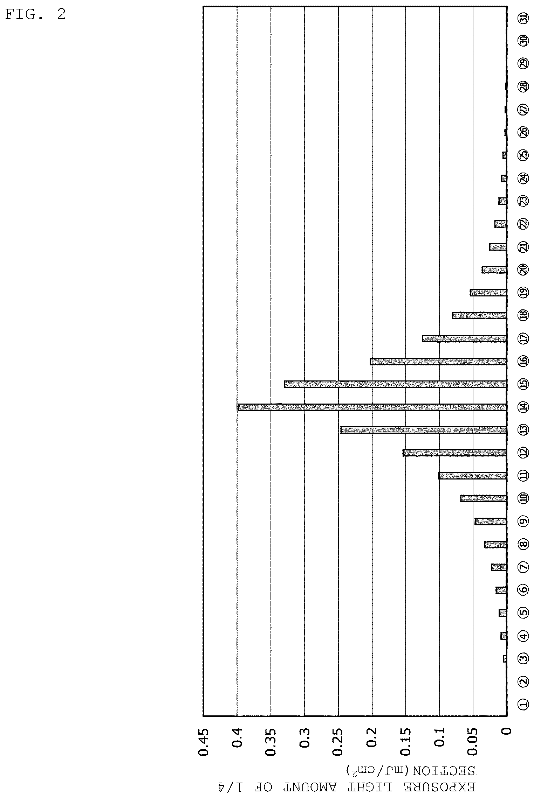

The exposure light amount determined for each light receiving surface by the calculation described above is shown in FIG. 2 in the order of the light receiving surfaces.

It was found that the exposure light amount was the highest in the vicinity of the center where the ultraviolet light LED element serving as a light source was installed, and from there, it was attenuating toward both the suction port 11A side and the discharge port 11B side in accordance with the distance, and there was almost no light amount leaked out from the suction port 11A and the discharge port 11B.

### (Simulation of Integrated Exposure Light Amount in Fluid Cleaning Circulator)

Next, an integrated exposure light amount in the fluid cleaning circulator was determined by simulation.

As a result from integrating the exposure light amounts of the light receiving surfaces entering from the suction port 11A, the total integrated exposure light amount at the time point of the discharge port 11B was 2.027 mJ/cm².

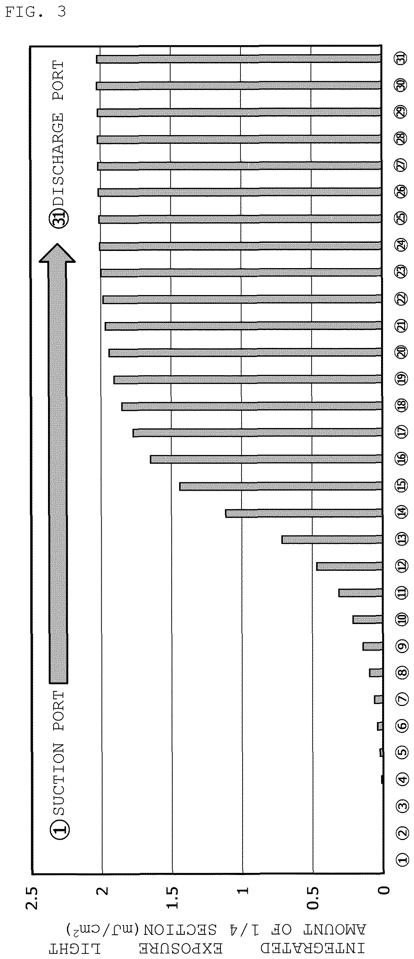

The integrated exposure light amount at the time point of reaching each light receiving surface by the calculation described above is shown in FIG. 3 in order from the inlet to the outlet.

Numerical values were obtained such that the integrated exposure light amount does not increase very much in the vicinity of the suction port 11A but the integrated value does increase sharply in the vicinity of the center near the light source, and then saturated. It was found that the total integrated exposure light amount of 2.027 mJ/cm² using the ultraviolet light of 275 nm having a high deactivating effect has a sufficient ability of deactivating the COVID-19 viruses in comparison to values of the other test examples.

### Industrial Applicability

Regarding handling of the COVID-19 viruses, it is highly difficult to perform an actual test due to the dangers of the viruses. However, in addition to the results of the test examples which have already been performed, as proved by the values calculated on the logical basis as physical numerical values, it is assured that the fluid cleaning circulator of the present invention exerts extremely excellent performances even in a case where the fluid cleaning circulator is actually used for cleaning similar types of viruses, etc.

## Claims

1. A fluid cleaning circulator that deactivates harmful microorganisms in a fluid by ultraviolet light irradiation, wherein
a hollow circular pipe formed in a spiral shape inside a closed container is provided, both ends of the hollow circular pipe are fixed to an inner wall of the closed container, and holes for a fluid inlet and a fluid outlet for allowing the fluid to flow through the hollow circular pipe are formed in the closed container,
surface processing that reflects ultraviolet light is applied to an inner surface of the hollow circular pipe, and a radiation prevention means that prevents radiation of an ultraviolet light from the fluid inlet and the fluid outlet to the outside is provided,
an ultraviolet light LED element is provided on an inner wall of the hollow circular pipe only within a distance of approximately 1/2 of a flow passage length of the hollow circular pipe from an end portion of the hollow circular pipe on the fluid inlet side, and
an interior of the hollow circular pipe is irradiated by light emission by the ultraviolet light LED element.

2. The fluid cleaning circulator according to Claim 1, wherein
the ultraviolet light to be irradiated is UV-C, and has a wavelength of 250 to 280 nm.
